(19) Europäisches Patentamt
European Patent Office
Office européen des brevets

(11) Veröffentlichungsnummer : **0 063 276**
**B1**

(12) # EUROPÄISCHE PATENTSCHRIFT

(45) Veröffentlichungstag der Patentschrift : **13.06.84**

(51) Int. Cl.³ : **C 09 B 62/513**

(21) Anmeldenummer : **82102762.0**

(22) Anmeldetag : **01.04.82**

(54) **Verfahren zur Herstellung von Disazoverbindungen.**

(30) Priorität : **07.04.81 DE 3113885**

(43) Veröffentlichungstag der Anmeldung : **27.10.82 Patentblatt 82/43**

(45) Bekanntmachung des Hinweises auf die Patenterteilung : **13.06.84 Patentblatt 84/24**

(84) Benannte Vertragsstaaten : **BE CH DE FR GB IT LI**

(56) Entgegenhaltungen :
**DE-C- 960 534**
**JP-A-72 014 348**

(73) Patentinhaber : **HOECHST AKTIENGESELLSCHAFT**
**Postfach 80 03 20**
**D-6230 Frankfurt am Main 80 (DE)**

(72) Erfinder : **Hoyer, Ernst, Dr.**
**Eptingweg 3**
**D-6230 Frankfurt am Main 80 (DE)**
Erfinder : **Fass, Rudolf**
**Memelstrasse 28**
**D-6233 Kelkheim (Taunus) (DE)**

EP 0 063 276 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung liegt auf dem technischen Gebiet der Synthese von Disazofarbstoffen.

Aus der Literatur and Disazoverbindungen aus zwei Diazokomponenten und einer bivalenten Kupplungskomponente bekannt, deren bivalente Kupplungskomponente die 1-Amino-8-hydroxy-3,6-disulfonsäure ist und deren eine oder beide Diazokomponenten eine β-Sulfatoäthylsulfonyl-Gruppe oder Vinylsulfonyl-Gruppe als faserreaktive Reste enthalten. Sie werden durch zweiseitiges Ankuppeln von 1-Amino-8-hydroxy-3,6-disulfonsäure mit zwei aromatischen Aminen als Diazokomponenten, von denen mindestens eine einen der obengenannten faserreaktiven Reste enthält, hergestellt, und zwar auf die Weise, daß zuerst eine dieser Diazokomponenten im stärker sauren Bereich auf diese Kupplungskomponente gekuppelt wird und die erhaltene Monoazoverbindung unter Zugabe der zweiten Diazokomponente im schwach sauren bis schwach alkalischen Bereich mit der zweiten Diazokomponente zur Disazoverbindung gekuppelt wird. In allen diesen Fällen werden somit die Kupplungen stufenweise nacheinander durchgeführt. Bei dieser zweifachen, stufenweisen Kupplung wird im ersten Kupplungsschritt die erste Azobrücke in ortho-Stellung zur Aminogruppe der Kupplungskomponente eingeführt und anschließend in diese Monoazoverbindung die zweite Azogruppe in ortho-Stellung zur Hydroxygruppe der Kupplungskomponente. Solche Synthesen der zweifachen, stufenweisen Kupplung zur Herstellung von Disazoverbindungen sind aus der deutschen Offenlegungsschrift 16 44 198, der deutschen Patentschrift 960 534 und den japanischen Patentbekanntmachungen Sho-43-15299 und Sho-47-14348 bekannt.

Es wurde nun gefunden, daß man überraschenderweise solche Disazoverbindungen mit bestimmter Konstitution und mit sehr guten anwendungstechnischen Eigenschaften und sehr guten Echtheitseigenschaften ebenfalls erhält, wenn man zur Synthese dieser bestimmten Disazoverbindungen die Diazoniumsalze der beiden als Ausgangsverbindungen dienenden Diazokomponenten und die als Ausgangsverbindung dienende bivalente Kupplungskomponente nicht stufenweise ein- und miteinander umsetzt, sondern im Gemisch miteinander kuppelt.

Die vorliegende Erfindung betrifft deshalb ein Verfahren zur Herstellung von Disazoverbindungen der allgemeinen Formel (1)

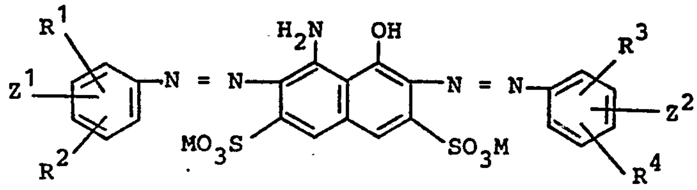

(1)

in welcher $R^1$ ein Wasserstoffatom, ein Chlor- oder Bromatom oder eine Sulfogruppe bedeutet, $R^2$ ein Wasserstoffatom oder ein Chlor- oder ein Bromatom ist, $R^3$ für ein Wasserstoffatom, eine Alkylgruppe von 1 bis 4 C-Atomen, wie die Äthyl- und bevorzugt die Methylgruppe, oder für eine Alkoxygruppe von 1 bis 4 C-Atomen, wie die Äthoxy- und bevorzugt die Methoxygruppe, steht und $R^4$ eine Alkoxygruppe von 1 bis 4 C-Atomen, wie die Äthoxy- und bevorzugt die Methoxygruppe, bedeutet sowie $Z^1$ und $Z^2$ jedes im Benzolkern in meta- oder para-Stellung zur Azogruppe gebunden ist und jedes für ein Wasserstoffatom oder die β-Thiosulfatoäthylsulfonyl-Gruppe (entsprechend der Formel $-SO_2-CH_2-CH_2-S-SO_3M$ mit M der nachstehend angegebenen Bedeutung) oder für die Vinylsulfonyl-Gruppe oder für die β-Sulfatoäthylsulfonyl-Gruppe (entsprechend der Formel $-SO_2-CH_2-CH_2-OSO_3M$ mit M der nachstehend angegebenen Bedeutung) steht, wobei $Z^1$ und $Z^2$ beide zueinander gleich oder voneinander verschieden sein können, jedoch mit der Maßgabe, daß beide nicht gleichzeitig für ein Wasserstoffatom stehen, und M ein Wasserstoffatom oder das Äquivalent eines Metalls, vorzugsweise eines Alkali- oder Erdalkalimetalls, wie insbesondere des Natriums, Kaliums und Calciums, darstellt und die Formelreste $R^1$, $R^2$, $R^3$, $R^4$ und M zueinander gleich oder voneinander verschieden sowie zu $Z^1$ und $Z^2$ gleich oder zueinander verschieden sind, indem man ein Gemisch zweier diazotierter aromatischer Amine der allgemeinen Formeln (2) und (3)

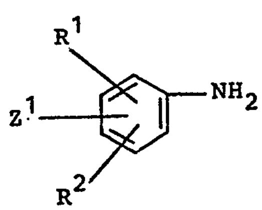

(2)

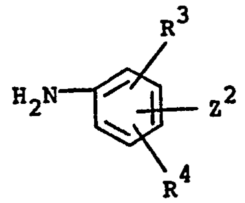

(3)

in welchen $R^1$, $R^2$, $R^3$, $R^4$, $Z^1$ und $Z^2$ die obengenannten Bedeutungen haben, mit einer Kupplungskomponente der allgemeinen Formel (4)

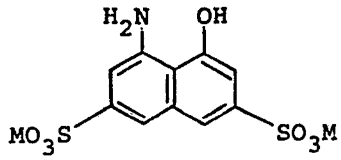

(4)

mit M der obengenannten Bedeutung zuerst im stark sauren Bereich und sodann im schwach sauren bis schwach alkalischen Bereich umsetzt. Die Umsetzung erfolgt mit etwa äquimolaren Mengen der Ausgangsverbindungen.

Bei dem im schwach alkalischen Bereich durchgeführten Reaktionsschritt können Anteile von β-Sulfatoäthylsulfonyl- oder β-Thiosulfatoäthylsulfonyl-Gruppen in Vinylsulfonyl-Gruppen übergeführt werden.

Gemische von beiden diazotierten Aminen der Formeln (2) und (3) erhält man, indem man beispielsweise jedes der beiden aromatischen Amine getrennt in an und für sich üblicher Weise diazotiert und anschließend die beiden Diazoniumsalze miteinander vermischt und mit der Kupplungskomponente vereinigt oder indem man ein Gemisch beider aromatischen Amine gemeinsam diazotiert und das Gemisch dieser Diazoniumsalze mit der Kupplungskomponente vereinigt.

Die im stark sauren, bevorzugt wäßrigen Medium zuerst erfolgende Umsetzung wird bevorzugt in einem pH-Bereich zwischen 0 und 3, insbesondere zwischen 0,5 und 2,5, durchgeführt. Die Kupplungstemperatur kann im Bereich zwischen 0 °C und 30 °C gewählt werden ; bevorzugt liegt sie zwischen 5 und 25 °C. Die Kupplung wird sodann im schwach sauren bis schwach alkalischen Medium weitergeführt ; als pH kann ein Wert zwischen 4 und 8 gewählt werden. Bevorzugt wird die Umsetzung bei einem pH-Wert zwischen 4,5 und 7 durchgeführt. Auch hier kann die Reaktionstemperatur in einem Bereich zwischen 0 und 30 °C gewählt werden ; bevorzugt führt man die Kupplung jedoch bei einer Temperatur zwischen 5 und 25 °C durch.

Das erfindungsgemäße Verfahren kann beispielsweise folgendermaßen ausgeführt werden : Man vermischt die wäßrigen Lösungen oder Suspensionen bzw. die Lösung oder die Suspension der Diazoniumsalze beider aromatischen Amine des Formeln (2) und (3) mit einer wäßrigen Lösung oder Suspension der Kupplungskomponente der Formel (4) und stellt erforderlichenfalls einen stark sauren, vorzugsweise salzsauren, pH-Bereich, insbesondere bevorzugt einen pH-Wert zwischen 0 und 3 ein. Man rührt bei einer Temperatur zwischen 0 und 30 °C, vorzugsweise 5 und 25 °C, mehrere Stunden, wie 4 bis 12 Stunden, und stellt sodann mittels eines säurebindenden Mittels einen schwach sauren bis schwach alkalischen pH-Bereich ein, wobei der pH-Wert bevorzugt zwischen 4,5 und 7 gewählt wird. Man rührt in diesem pH-Bereich bei einer Temperatur zwischen 0 und 30 °C, bevorzugt 5 und 25 °C, wenige Stunden, wie 2 bis 6 Stunden, weiter und isoliert sodann die gebildete Disazoverbindung entsprechend der allgemeinen Formel (1) auf übliche Weise.

Als säurebindende Mittel zur Einstellung des schwach sauren bis schwach alkalischen pH-Bereiches während der Kupplungsreaktion werden vorzugsweise die Alkali- oder Erdalkalimetallsalze von schwachen anorganischen oder organischen Säuren, so der Kohlensäure, der Essigsäure, der Borsäure, der Oxalsäure, oder die sauren oder basischen Salze der Phosphorsäure verwendet ; die Alkali- und Erdalkalimetallsalze sind bevorzugt die Natrium-, Kalium- und Calciumsalze. Insbesondere eignen sich als säurebindende Mittel zur Abstumpfung des stark sauren pH-Bereiches Natrium- und Kaliumcarbonat, Natriumacetat, Natriumborat und besonders vorteilhaft Natriumhydrogencarbonat.

Die Isolierung der erfindungsgemäß hergestellten Verbindungen aus ihren Syntheseansätzen geschieht nach allgemein bekannten Methoden, so beispielsweise durch Ausfällen der Disazoverbindungen aus dem Reaktionsmedium mittels eines Elektrolyten, wie beispielsweise Natriumchlorid oder Kaliumchlorid oder aber durch Eindampfen der Reaktionslösung selbst, beispielsweise durch Sprühtrocknung. Der Syntheseansatz kann aber auch selbst, falls gewünscht, gegebenenfalls nach Aufkonzentrierung und/oder Zusatz von Puffersubstanzen-, direkt als Flüssigpräparation weiterverwendet werden.

Die erfindungsgemäß hergestellten Disazoverbindungen der allgemeinen Formel (1) stellen Farbstoffe mit sehr guten faserreaktiven und sehr guten anwendungstechnischen Eigenschaften dar, die analog bekannten Applikations- und Fixiermethoden auf hydroxy- und/oder carbonamidgruppenhaltigen Fasermaterialien farbstarke, marineblaue bis blaustichig schwarze, grünstichig schwarze, rotstichig schwarze und reinschwarze Färbungen und Drucke mit guten bis sehr guten Echtheitseigenschaften liefern. Als solche Echteiten können hervorgehoben werden die Lichtechtheit, die Waschechtheiten, die Schweißechtheiten, die Säure- und Alkaliechtheiten sowie die Überfärbe-, Meerwasser- und Wasserechtheiten. Der Farbaufbau der erfindungsgemäß hergestellten Disazoverbindungen ist ausgezeichnet ; ebenso liegen die Fixiergrade entsprechend den verschiedenen Applikationsverfahren außerordentlich hoch. Hydroxygruppenhaltige Fasermaterialien, mit denen die erfindungsgemäß hergestellten Verbindungen vorteilhaft gefärbt werden können, sind insbesondere Cellulosefasermaterialien, wie Baumwolle. Carbonamidgruppenhaltige Fasermaterialien sind insbesondere Wolle und Seide und synthetische Polyamidfasern, wie aus Polyamid-6 oder Polyamid-6,6.

Die nach dem erfindungsgemäßen Verfahren hergestellten Disazoverbindungen sind konstitutionsmäßig mit den entsprechenden Disazoverbindungen der allgemeinen Formel (1) identisch, die nach dem bisher üblichen und bekannten Syntheseweg durch stufenweise Kupplung der beiden Diazokomponenten mit der Kupplungskomponente der Formel (4) unter Zugabe zunächst der Diazoniumsalzverbindung der aromatischen Amins der allgemeinen Formel (2) im sauren pH-Bereich und sodann unter Zugabe der Diazoniumsalzverbindung des aromatischen Amins der Formel (3) im schwach sauren bis schwach alkalischen Bereich synthetisiert wurden. Dementsprechend sind auch die Farbstoffeigenschaften der erfindungsgemäß hergestellten Disazoverbindungen mit denen der nach dem bisher bekannten und üblichen Verfahren der stufenweisen Kupplung hergestellten Disazoverbindungen der allgemeinen Formel (1) identisch. Es überrascht, daß mit dem erfindungsgemäßen Verfahren eine einheitliche Disazoverbindung erhalten wird. Dies war nicht vorauszusehen; vielmehr mußte erwartet werden, daß man beim Einsatz des Gemisches der beiden Diazokomponenten mit der Kupplungskomponente ein Gemisch von Disazoverbindungen erhält, und zwar ein Gemisch von 4 Disazoverbindungen, von denen die eine die Verbindung der allgemeinen Formel (1) und eine andere deren isomere Verbindung ist, in welcher die Diazokomponente der Formel (3) o-ständig zur Aminogruppe und die Diazokomponente der Formel (2) o-ständig zur Hydroxygruppe gekuppelt hat, sowie zwei weitere hiervon Disazoverbindungen sind, in welchen die beiden Diazokomponenten jeweils identisch sind, d. h. bei der einen die Diazokomponenten die der allgemeinen Formel (2), bei der anderen die Diazokomponenten die der allgemeinen Formel (3) sind.

Das erfindungsgemäße Verfahren hat gegenüber den bisher bekannten und üblichen Verfahrensweisen zur Herstellung der Verbindungen der allgemeinen Formel (1) den Vorteil, daß es als Eintopf-Verfahren durchgeführt werden kann. Die beiden aromatischen Amine können gemeinsam diazotiert werden, und die Kupplung kann im gleichen Gefäß erfolgen. Der apparative und arbeitszeitmäßige Aufwand zur Herstellung der Disazoverbindungen wird somit deutlich vermindert, was in der Technik einen erheblichen Vorteil mit sich bringt.

Bevorzugt ist das erfindungsgemäße Verfahren zur Herstellung von Disazoverbindungen der allgemeinen Formel (1), in welcher $R^1$ für ein Chlor- oder Bromatom oder eine Sulfogruppe steht, $R^2$ ein Wasserstoffatom ist, $R^3$ und $R^4$ beide gleich sind und jedes eine Methoxy- oder Äthoxygruppe bedeutet und $Z^1$ und $Z^2$ beide zueinander gleich oder voneinander verschieden sind und jedes für eine β-Thiosulfatoäthylsulfonyl-, β-Sulfatoäthylsulfonyl- oder Vinylsulfonylgruppe steht, beide bevorzugt jedoch die β-Sulfatoäthylsulfonyl-Gruppe sind, wobei $Z^1$ und $Z^2$ beide bevorzugt jeweils in para-Stellung zu der Azogruppe gebunden sind. Insbesondere bevorzugt ist die Herstellung der Verbindungen der allgemeinen Formel (1), in welcher $R^1$ und $R^2$ beide ein Wasserstoffatom darstellen, $R^3$ und $R^4$ beide gleich sind und jedes für die Äthoxygruppe, bevorzugt die Methoxygruppe steht und $Z^1$ und $Z^2$ die obengenannten bevorzugten Bedeutungen haben, insbesondere bevorzugt jedoch beide die gleiche Bedeutung besitzen und jedes für die β-Sulfatoäthylsulfonyl-Gruppe steht und bevorzugt in para-Stellung zur Azogruppe gebunden ist.

Die nachfolgenden Beispiele dienen zur Erläuterung der Erfindung. Die Teile sind Gewichtsteile, die Angaben in Prozent beziehen sich auf Gewichtsprozent, sofern nichts anderes vermerkt. Volumenteile verhalten sich zu Gewichtsteilen wie Liter zu Kilogramm.

### Beispiel 1

70,25 Teile 4-β-Sulfatoäthylsulfonyl-anilin und 85,25 Teile 4-β-Sulfatoäthylsulfonyl-2,5-dimethoxy-anilin werden in 675 Teile Wasser eingetragen und bei 20 bis 22 °C innerhalb einer Stunde mittels Natriumbicarbonat auf einen pH-Wert von 5,3 bis 6,0 eingestellt; hierbei gehen die Aniline in Lösung. Sodann werden 107,5 Volumenteile einer 31 %igen wäßrigen Salzsäure und 475 Teile Eis zugegeben, um die Lösung kongosauer und auf eine Temperatur von 0 bis 5 °C zu stellen. Bei dieser Temperatur wird die Diazotierung mittels 65,5 Volumenteilen einer 40 %igen wäßrigen Natriumnitritlösung durchgeführt; anschließend wird ein geringer Überschuß an salpetriger Säure mit 0,5 Teilen Amidosulfonsäure zerstört. Sodann werden 77,8 Teile 1-Amino-8-hydroxynaphthalin-3,6-disulfonsäure zugegeben und der Ansatz 4 bis 5 Stunden bei einer Temperatur zwischen 4 und 8 °C und einem pH-Wert zwischen 1 und 2 gerührt. Nach dieser Reaktionszeit trägt man innerhalb von 90 Minuten 66,7 Teile Natriumbicarbonat ein, um den Ansatz auf einen pH-Wert von 6 zu stellen; es wird noch etwa 4 bis 5 Stunden nachgerührt, um die Bildung der Disazoverbindung zu vervollständigen; zu ihrer Isolierung wird die Syntheselösung sodann sprühgetrocknet. Es wird ein schwarzes, elektrolythaltiges (vorwiegend Natriumchlorid) Pulver erhalten, das 249 Teile des Natriumsalzes der Disazoverbindung der Formel

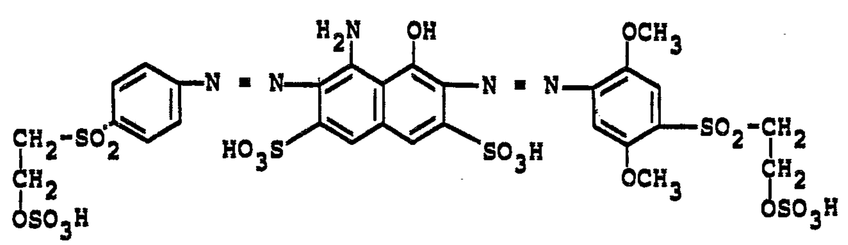

enthält. Diese Verbindung zeigt sehr gute faserreaktive Farbstoffeigenschaften und liefert auf den für faserreaktive Farbstoffe üblichen und bekannten Applikations- und Fixiermethoden auf den in der Beschreibung genannten Materialien, insbesondere auf Cellulosefasermaterialien, grünstichig schwarze Färbungen mit sehr guten Echtheiten, die beispielsweise in der Beschreibung genannt sind.

Beispiele 2 bis 21

In den nachfolgenden Tabellenbeispielen 2 bis 21 werden weitere Disazoverbindungen entsprechend der allgemeinen Formel (1) beschrieben, die einheitlich in erfindungsgemäßer Weise durch Umsetzung eines Gemisches der entsprechenden, dort angegebenen diazotierten Amine der allgemeinen Formeln (2) und (3) mit 1-Amino-8-hydroxy-3,6-disulfonsäure als bivalenter Kupplungskomponente, beispielsweise analog der im Beispiel 1 angegebenen Verfahrensweise, hergestellt werden können. Die in der Tabelle formelmäßig angegebenen Verbindungen (1) sind in Form der freien Säure geschrieben ; sie werden gemäß der Verfahrensweise des Beispieles 1 als deren Natriumsalze erhalten. Sie stellen faserreaktive Farbstoffe mit guten anwendungstechnischen Eigenschaften und guten Echtheiten dar, die beispielsweise Cellulosefasermaterialien in der im jeweiligen Tabellenbeispiel angegebenen Nuance färben.

(Siehe die Tabelle Seite 6 ff.)

| Bsp. | Amin der Formel (2) | Amin der Formel (3) | Disazoverbindung der Formel (1) | Farbton |
|---|---|---|---|---|
| 2 | 2-Brom-4-ß-sulfato-äthylsulfonyl-anilin | 2,5-Dimethoxy-4-ß-sulfatoäthylsulfonyl-anilin | 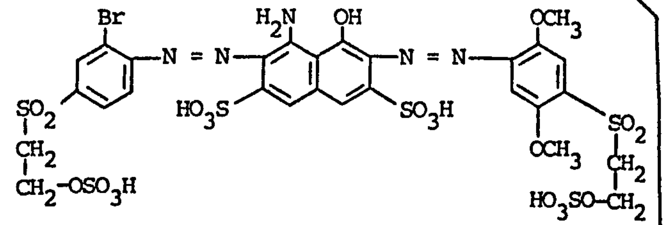 | grün-stichig schwarz |
| 3 | dito | 2-Methoxy-5-methyl-4-ß-sulfatoäthylsulfonyl-anilin | 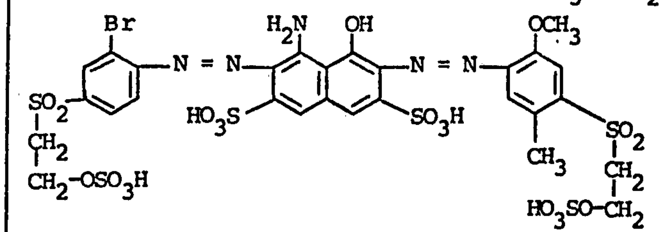 | grün-stichig schwarz |
| 4 | 2-Chlor-4-ß-sulfato-äthylsulfonyl-anilin | 2-Methoxy-4-ß-sulfato-äthylsulfonyl-anilin | 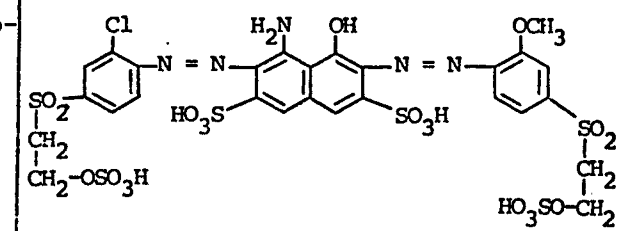 | grün-stichig schwarz |
| 5 | 4-ß-Sulfatoäthylsulfonyl-anilin | 2-Methoxy-5-methyl-4-ß-sulfatoäthylsulfonyl-anilin | 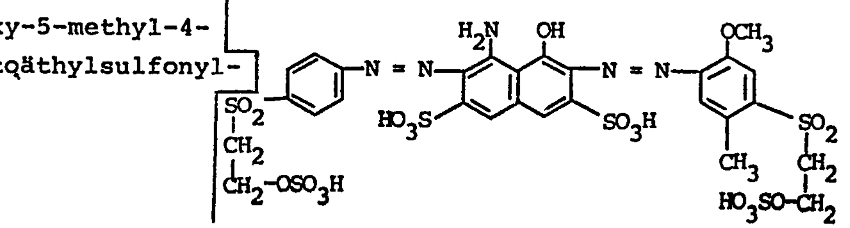 | marine-blau |

| Bsp. | Amin der Formel (2) | Amin der Formel (3) | Disazoverbindung der Formel (1) | Farbton |
|---|---|---|---|---|
| 6 | 4-ß-Sulfatoäthyl-sulfonyl-anilin | 2-Methoxy-5-ß-sulfato-äthylsulfonyl-anilin | 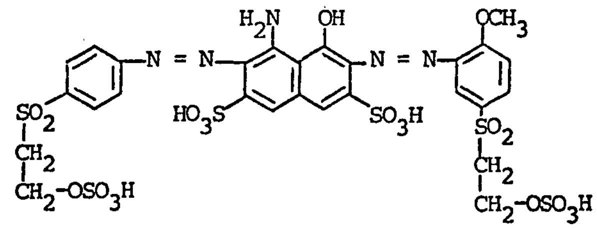 | marineblau |
| 7 | 2-Brom-4-ß-sulfato-äthylsulfonyl-anilin | dito | 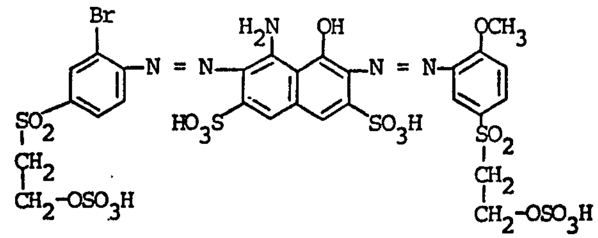 | marineblau |
| 8 | dito | 4-Methoxy-3-ß-sulfa-toäthylsulfonyl-anilin | 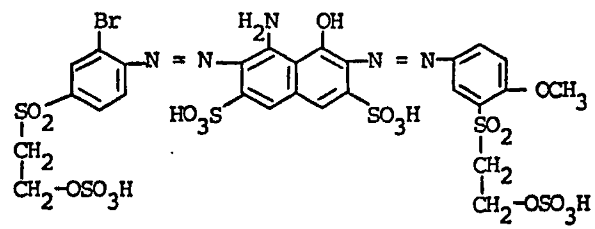 | schwarz |
| 9 | 2,6-Dichlor-4-ß-sulfatoäthylsul-fonyl-anilin | 2-Methoxy-5-methyl-4-ß-sulfatoäthyl-sulfonyl-anilin | 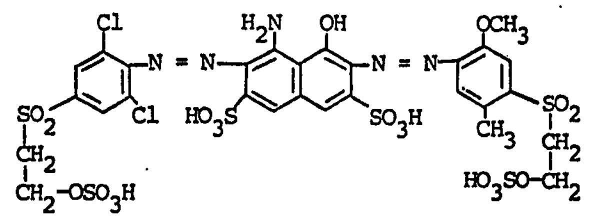 | grünstichig schwarz |

| Bsp. | Amin der Formel (2) | Amin der Formel (3) | Disazoverbindung der Formel (1) | Farbton |
|---|---|---|---|---|
| 10 | 2,6-Dibrom-4-ß-sulfatoäthylsulfonyl-anilin | 2-Methoxy-5-ß-sulfatoäthylsulfonyl-anilin | 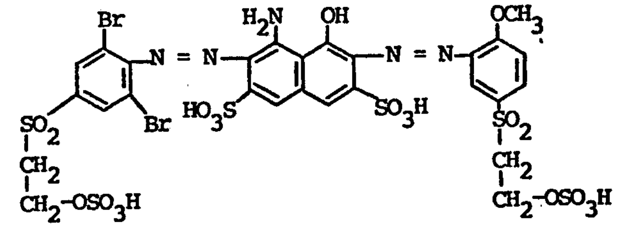 | schwarz |
| 11 | 2-Chlor-5-ß-sulfatoäthylsulfonyl-anilin | dito | 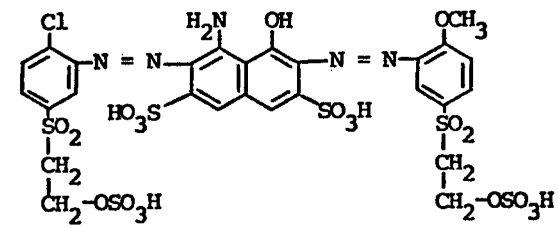 | schwarz |
| 12 | 4-ß-Sulfatoäthylsulfonyl-anilin-2-sulfonsäure | 2,5-Dimethoxy-4-ß-sulfatoäthylsulfonyl-anilin | 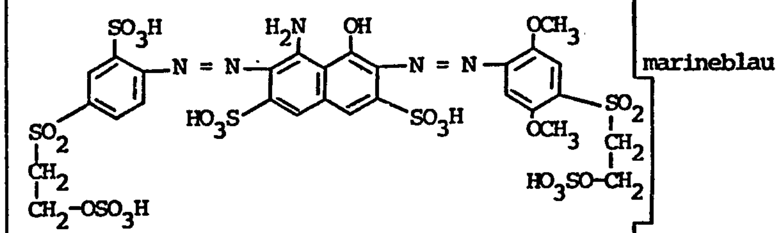 | marineblau |
| 13 | dito | 2-Methoxy-4-ß-sulfatoäthylsulfonyl-anilin | 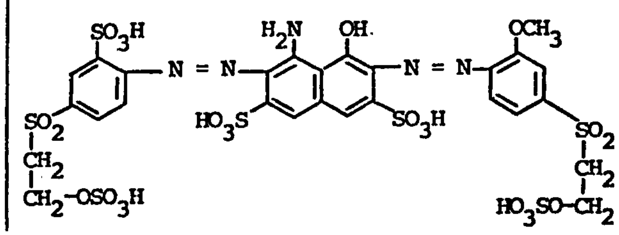 | marineblau |

| Bsp. | Amin der Formel (2) | Amin der Formel (3) | Disazoverbindung der Formel (1) | Farbton |
|---|---|---|---|---|
| 14 | 4-Vinylsulfonyl-anilin | 2,5-Dimethoxy-4-vinylsulfonyl-anilin | 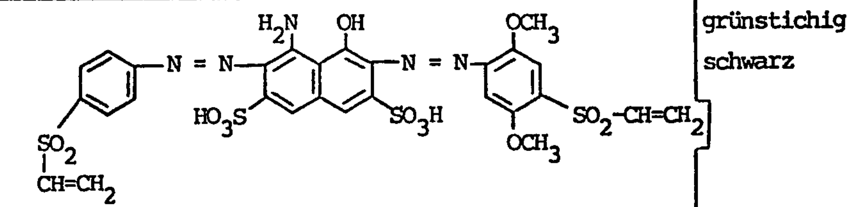 | grünstichig schwarz |
| 15 | 4-Vinylsulfonyl-anilin-2-sulfonsäure | 2-Methoxy-5-methyl-4-vinyl-sulfonyl-anilin | 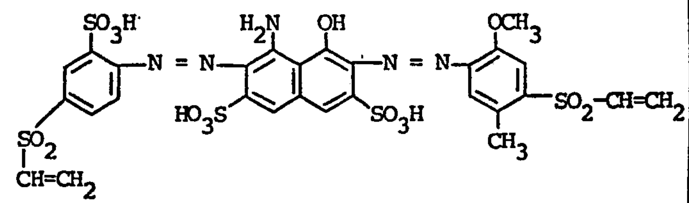 | marineblau |
| 16 | Anilin-4-sulfonsäure | 2,5-Dimethoxy-4-ß-sulfatoäthyl-sulfonyl-anilin | 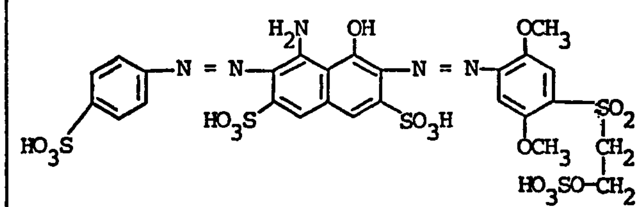 | grünstichig schwarz |
| 17 | 4-Chlor-anilin-2-sulfonsäure | dito | 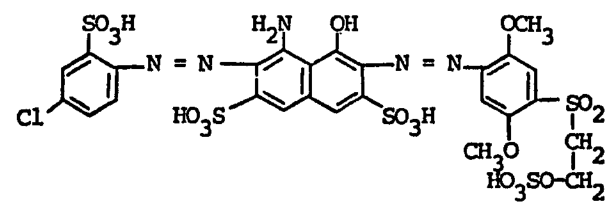 | grünstichig schwarz |

| Bsp. | Amin der Formel (2) | Amin der Formel (3) | Disazoverbindung der Formel (1) | Farbton |
|---|---|---|---|---|
| 18 | 2-Brom-4-ß-sulfato-äthylsulfonyl-anilin | 2-Methoxy-4-ß-sulfa-toäthylsulfonyl-anilin | 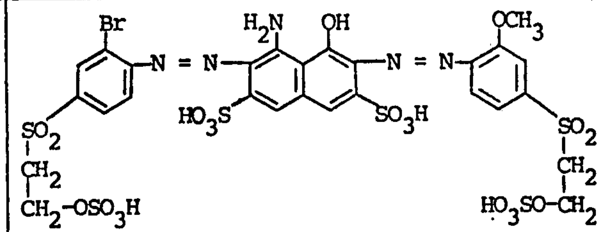 | schwarz |
| 19 | Anilin-4-sulfon-säure | 2-Methoxy-5-methyl-4-ß-sulfatoäthyl-sulfonyl-anilin | 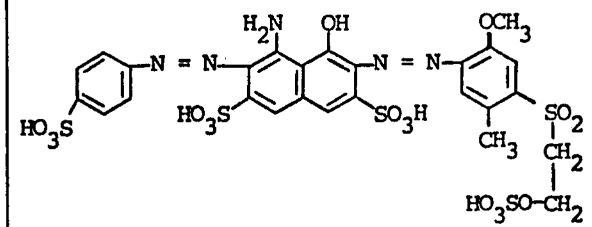 | marineblau |
| 20 | Anilin-2-sulfon-säure | 2,5-Dimethoxy-4-ß-sulfatoäthylsul-fonyl-anilin | 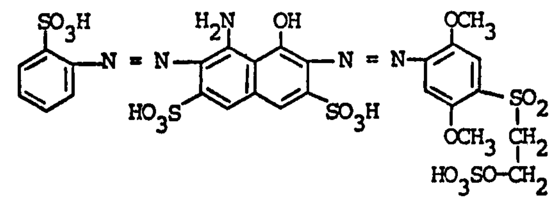 | marineblau |
| 21 | Anilin-4-sulfon-säure | 2-Methoxy-5-ß-sul-fatoäthylsulfonyl-anilin | 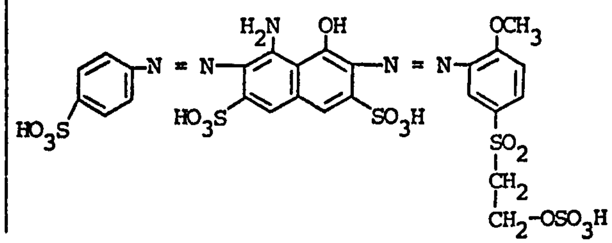 | marineblau |

**Ansprüche**

1. Verfahren zur Herstellung von Disazoverbindungen der allgemeinen Formel (1)

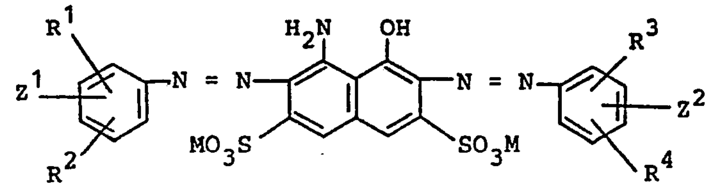

(1)

in welcher $R^1$ ein Wasserstoffatom, ein Chlor- oder Bromatom oder eine Sulfogruppe bedeutet, $R^2$ ein Wasserstoffatom oder ein Chlor- oder ein Bromatom ist, $R^3$ für ein Wasserstoffatom, eine Alkylgruppe von 1 bis 4 C-Atomen oder eine Alkoxygruppe von 1 bis 4 C-Atomen steht und $R^4$ eine Alkoxygruppe von 1 bis 4 C-Atomen bedeutet sowie $Z^1$ und $Z^2$ jedes im Benzolkern in meta- oder para-Stellung zur Azogruppe gebunden ist und jedes für ein Wasserstoffatom, die β-Thiosulfatoäthylsulfonyl-Gruppe, die Vinylsulfonyl-Gruppe oder die β-Sulfatoäthylsulfonyl-Gruppe steht, wobei $Z^1$ und $Z^2$ beide zueinander gleich oder voneinander verschieden sein können, jedoch mit der Maßgabe, daß beide nicht gleichzeitig für ein Wasserstoffatom stehen, und M ein Wasserstoffatom oder das Äquivalent eines Metalls darstellt und die Formelreste $R^1$, $R^2$, $R^3$, $R^4$ und M zueinander gleich oder voneinander verschieden sowie zu $Z^1$ und $Z^2$ gleich oder zueinander verschieden sind, dadurch gekennzeichnet, daß man ein Gemisch zweier diazotierter aromatischer Amine der allgemeinen Formeln (2) und (3)

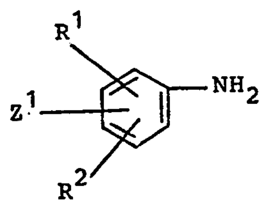

(2)

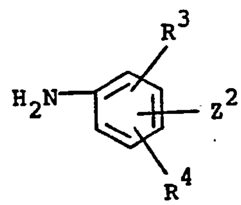

(3)

in welchen $R^1$, $R^2$, $R^3$, $R^4$, $Z^1$ und $Z^2$ die obengenannten Bedeutungen haben, mit einer Kupplungskomponente der allgemeinen Formel (4)

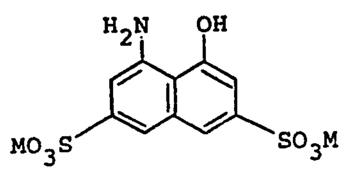

(4)

mit M der obengenannten Bedeutung zuerst im stark sauren Bereich und sodann im schwach sauren bis schwach alkalischen Bereich umsetzt.

2. Verfahren nach Anspruch 1, dadurch gekennzeichnet, daß in den dort angegebenen allgemeinen Formeln (1), (2), (3) und (4) $R^1$ ein Chlor- oder Bromatom oder eine Sulfogruppe bedeutet, $R^2$ ein Wasserstoffatom ist, $R^3$ und $R^4$ beide gleiche Bedeutungen besitzen und jedes eine Methoxy- oder Äthoxygruppe bedeutet, $Z^1$ und $Z^2$ zueinander gleich oder voneinander verschieden sind und jedes eine β-Thiosulfatoäthylsulfonyl-Gruppe, eine β-Sulfatoäthylsulfonyl-Gruppe oder die Vinylsulfonyl-Gruppe bedeutet und M die in Anspruch 1 genannte Bedeutung besitzt.

3. Verfahren nach Anspruch 1, dadurch gekennzeichnet, daß in den dort angegebenen allgemeinen Formeln (1), (2), (3) und (4) $R^1$ und $R^2$ beide ein Wasserstoffatom bedeuten, $R^3$ und $R^4$ beide gleich sind und jedes eine Methoxy- oder Äthoxygruppe darstellt, $Z^1$ und $Z^2$ zueinander gleich oder voneinander verschieden sind und jedes eine β-Thiosulfatoäthylsulfonyl- oder β-Sulfatoäthylsulfonyl- oder Vinylsulfonyl-Gruppe ist und M die in Anspruch 1 genannte Bedeutung besitzt.

4. Verfahren nach Anspruch 1, 2 oder 3, dadurch gekennzeichnet, daß $Z^1$ und $Z^2$ beide gleich sind und jedes für die β-Sulfatoäthylsulfonyl-Gruppe steht.

5. Verfahren nach Anspruch 2, 3 oder 4, dadurch gekennzeichnet, daß $Z^1$ und $Z^2$ jedes jeweils in para-Stellung zur Azogruppe gebunden ist.

6. Verfahren nach Anspruch 1, dadurch gekennzeichnet, daß man ein Gemisch zweier diazotierter aromatischer Amine der Formeln

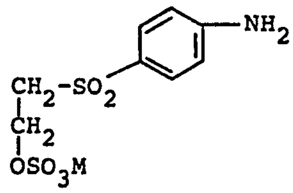

und

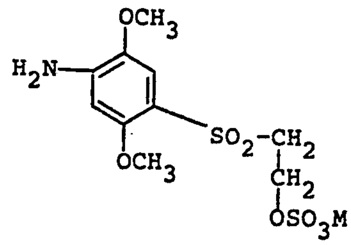

in welchen M die in Anspruch 1 genannten Bedeutungen besitzen, mit einer Verbindung der in Anspruch 1 genannten und definierten allgemeinen Formel (4) zur Verbindung der Formel

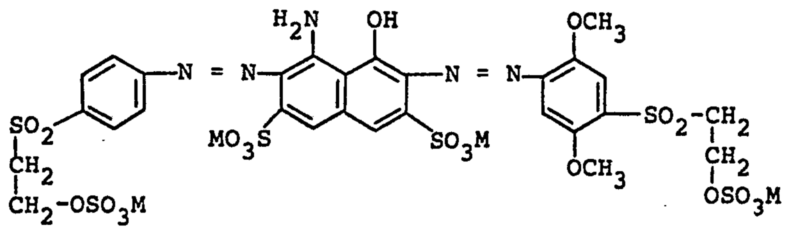

in welcher M die in Anspruch 1 genannte Bedeutung besitzt, kuppelt.

7. Verfahren nach einem der Ansprüche 1 bis 6, dadurch gekennzeichnet, daß M jeweils für Natrium oder Kalium steht.

**Claims**

1. A process for the preparation of disazo compounds of the general formula (1)

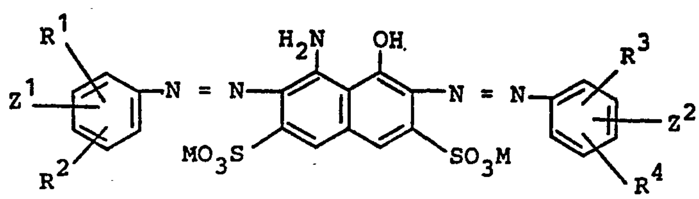

(1)

in which $R^1$ denotes a hydrogen atom, a chlorine or bromine atom or a sulfo group, $R^2$ is a hydrogen atom or a chlorine or bromine atom, $R^3$ represents a hydrogen atom, an alkyl group having 1 to 4 C-atoms or an alkoxy group having 1 to 4 C-atoms and $R^4$ denotes an alkoxy group having 1 to 4 C-atoms and $Z^1$ and $Z^2$ are each bonded in the benzene nucleus in the meta- or para-position relative to the azo group and each represents a hydrogen atom, the β-thiosulfatoethylsulfonyl group, the vinylsulfonyl group or the β-sulfatoethylsulfonyl group, and $Z^1$ and $Z^2$ can be identical to or different from one another, but with the proviso that they do not both represent a hydrogen atom at the same time, and M represents a hydrogen atom or the equivalent of a metal, and the formula moieties $R^1$, $R^2$, $R^3$, $R^4$ and M are identical to or different from one another and also identical to or different from $Z^1$ and $Z^2$, characterized by that a mixture of two diazotized aromatic amines of the general formulae (2) and (3)

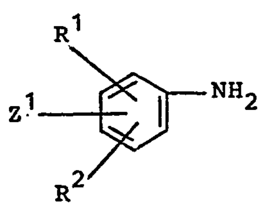

(2)

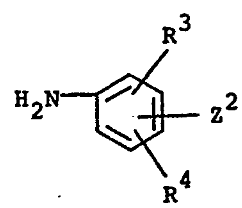

(3)

in which $R^1$, $R^2$, $R^3$, $R^4$, $Z^1$ and $Z^2$ have the abovementioned meanings, are reacted with a coupling component of the general formula (4)

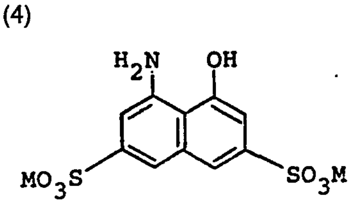

(4)

in which M has the abovementioned meaning, first in a strongly acid range and then in a weakly acid to weakly alkaline range.

2. The process according to claim 1, characterized in that, in the general formulae (1), (2), (3) and (4) indicated there, $R^1$ denotes a chlorine or bromine atom or a sulfo group, $R^2$ is a hydrogen atom, $R^3$ and $R^4$ have the same meaning and each denotes a methoxy or ethoxy group, $Z^1$ and $Z^2$ are identical to or different from one another and each denotes a β-thiosulfatoethylsulfonyl group, a β-sulfatoethylsulfonyl group or the vinylsulfonyl group and M has the meaning mentioned in claim 1.

3. The process according to claim 1, characterized in that, in the general formulae (1), (2), (3) and (4) indicated there, $R^1$ and $R^2$ both denote a hydrogen atom, $R^3$ and $R^4$ are identical and each represents a methoxy or ethoxy group, $Z^1$ and $Z^2$ are identical to or different from one another and each is a β-thiosulfatoethylsulfonyl or β-sulfatoethylsulfonyl or vinylsulfonyl group and M has the meaning mentioned in claim 1.

4. The process according to claim 1, 2 or 3, characterized in that $Z^1$ and $Z^2$ are identical and each represents the β-sulfatoethylsulfonyl group.

5. The process according to claim 2, 3 or 4, characterized in that $Z^1$ and $Z^2$ are each bonded in the para-position relative to the azo group.

6. The process according to claim 1, characterized in that a mixture of two diazotized aromatic amines of the formulae

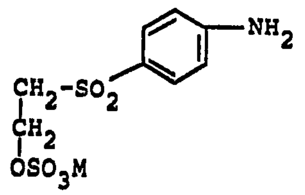

and

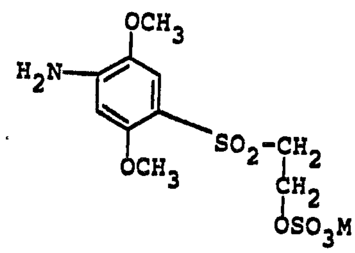

in which M has the meaning mentioned in claim 1, are coupled with a compound of the general formula (4) mentioned and defined in claim 1, to give a compound of the formula

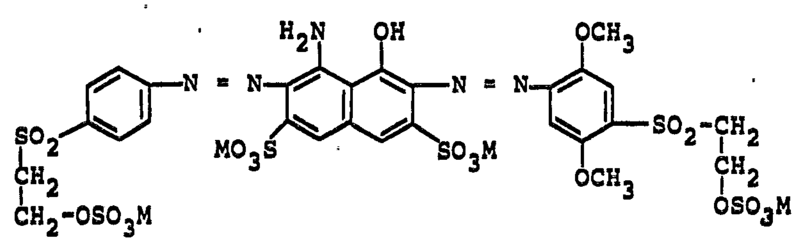

in which M has the meaning mentioned in claim 1.

7. The process according to any of claims 1 to 6, characterized in that M represents either sodium or potassium.

**Revendications**

1. Procédé de préparation de composés disazoïques répondant à la formule générale (1)

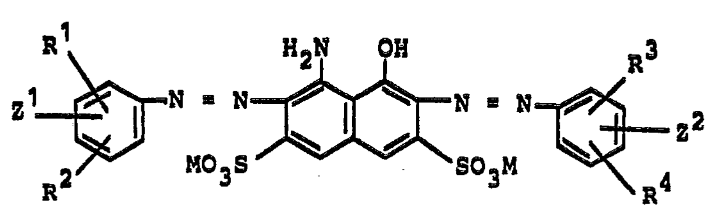

(1)

dans laquelle $R^1$ désigne un atome d'hydrogène, un atome de chlore ou de brome ou un groupe sulfo, $R^2$ représente un atome d'hydrogène ou un atome de chlore ou de brome, $R^3$ un atome d'hydrogène, un groupe alkyle en $C_1$ à $C_4$ ou un groupe alcoxy en $C_1$ à $C_4$ et $R^4$ désigne un groupe alcoxy en $C_1$ à $C_4$, et $Z^1$ et $Z^2$ sont liés chacun dans le noyau benzénique en position méta ou para par rapport au groupe azo, et représentent chacun un atome d'hydrogène, le groupe β-thiosulfatoéthylsulfonyle, le groupe vinylsulfonyle ou le groupe β-sulfatoéthylsulfonyle, $Z^1$ et $Z^2$ pouvant être identiques entre eux ou différents, sous réserve cependant que tous deux ne représentent pas simultanément un atome

d'hydrogène, et M représente un atome d'hydrogène ou l'équivalent d'un métal et les radicaux $R^1$, $R^2$, $R^3$, $R^4$ et M de la formule sont identiques entre eux ou différents, caractérisé en ce qu'on fait réagir un mélange de deux amines aromatiques diazotées répondant aux formules générales (2) et (3)

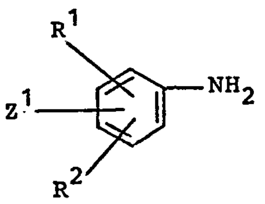 (2) 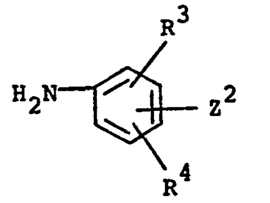 (3)

dans lesquelles $R^1$, $R^2$, $R^3$, $R^4$, $Z^1$ et $Z^2$ ont les significations indiquées ci-dessus, avec un constituant de copulation répondant à la formule générale (4)

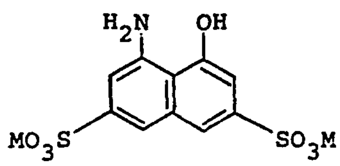 (4)

dans laquelle M a la signification indiquée ci-dessus, d'abord dans le domaine fortement acide, puis dans le domaine faiblement acide à faiblement alcalin.

2. Procédé suivant la revendication 1, caractérisé en ce que dans les formules générales (1), (2), (3) et (4) qui y sont indiquées, $R^1$ désigne un atome de chlore ou de brome ou un groupe sulfo, $R^2$ est un atome d'hydrogène, $R^3$ et $R^4$ ont les mêmes significations et désignent chacun un groupe méthoxy ou éthoxy, $Z^1$ et $Z^2$ sont identiques entre eux ou différents et désignent chacun un groupe β-thiosulfatoéthylsulfonyle, un groupe β-sulfatoéthylsulfonyle ou le groupe vinylsulfonyle et M a la signification indiquée dans la revendication 1.

3. Procédé suivant la revendication 1, caractérisé en ce que dans les formules générales (1), (2), (3) et (4) qui y sont indiquées, $R^1$ et $R^2$ désignent tous deux un atome d'hydrogène, $R^3$ et $R^4$ sont identiques et représentent chacun un groupe méthoxy ou éthoxy, $Z^1$ et $Z^2$ sont identiques ou différents et sont chacun un groupe β-thiosulfatoéthylsulfonyle ou β-sulfatoéthylsulfonyle ou vinylsulfonyle, et M a la signification indiquée dans la revendication 1.

4. Procédé suivant l'une quelconque des revendications 1, 2 ou 3, caractérisé en ce que $Z^1$ et $Z^2$ sont identiques et représentent chacun le groupe β-sulfatoéthylsulfonyle.

5. Procédé suivant l'une quelconque des revendications 2, 3 ou 4, caractérisé en ce que $Z^1$ et $Z^2$ sont liés chacun en position para par rapport au groupe azo.

6. Procédé suivant la revendication 1, caractérisé en ce qu'on copule un mélange de deux amines aromatiques diazotées répondant aux formules

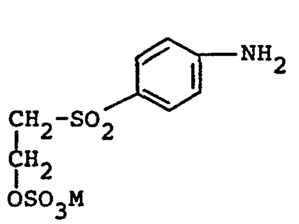 et 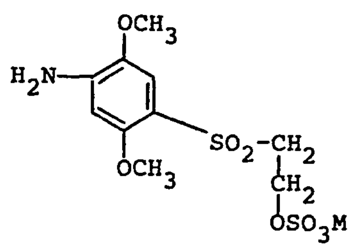

dans lesquelles M a les significations indiquées dans la revendication 1, avec un composé répondant à la formule générale (4) indiquée et définie dans la revendication 1, pour donner un composé répondant à la formule

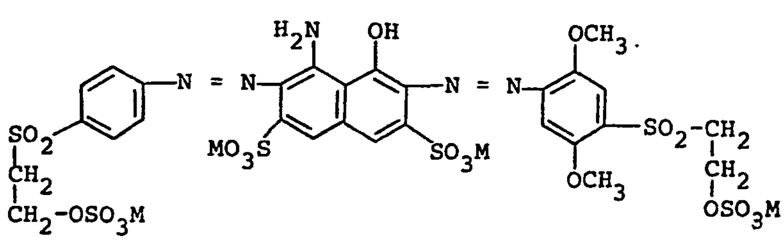

dans laquelle M a la signification indiquée dans la revendication 1.

7. Procédé suivant l'une quelconque des revendications 1 à 6, caractérisé en ce que M désigne à chaque fois le sodium ou le potassium.